# EUROPÄISCHE PATENTSCHRIFT

(11) **EP 0 704 426 B1**
(45) Veröffentlichungstag und Bekanntmachung des Hinweises auf die Patenterteilung: **06.05.1998**
(21) Anmeldenummer: 95114635.6
(22) Anmeldetag: 18.09.1995
(51) Int. Cl.: C07C 201/16, C07C 205/44

(54) **Verfahren zur Trennung von Nitrobenzaldehyd-Isomerengemischen**
Process for the separation of mixtures of nitrobenzaldehyde isomers
Procédé de séparation de mélanges d'isomère du nitrobenzaldehyde

(30) Priorität: 29.09.1994 DE 4434848
(43) Veröffentlichungstag der Anmeldung: 03.04.1996
(73) Patentinhaber: BAYER AG, 51368 Leverkusen (DE)
(72) Erfinder: Kissener, Wolfram, Dr., D-53819 Neunkirchen (DE); Emde, Herbert, Dr., D-51069 Köln (DE); Fessenbecker, Achim, Dr., D-76669 Bad Schönborn (DE)

(56) Entgegenhaltungen:
- EP-A- 0 091 575
- EP-A- 0 320 539
- DATABASE WPI Week 9143 Derwent Publications Ltd., London, GB; AN 91-312686 & ES-A-2 020 891 (ERCROS SA) , 1.Oktober 1991

## Beschreibung

Die vorliegende Erfindung betrifft ein vorteilhaftes Verfahren zur Trennung von Nitrobenzaldehyd-Isomerengemischen durch Destillation.

Bei der Herstellung von Nitrobenzaldehyd, z.B. durch Nitrierung von Benzaldehyd, fallen Gemische an, die 2-, 3- und 4-Nitrobenzaldehyd enthalten. Für die Weiterverwendung von Nitrobenzaldehyd, insbesondere zur Herstellung von pharmazeutischen Wirkstoffen, ist es erforderlich, möglichst isomerenfreie Produkte zur Verfügung zu haben, z.B. möglichst reinen 2-Nitrobenzaldehyd und 3-Nitrobenzaldehyd.

Eine Trennung von Nitrobenzaldehyd-Isomerengemischen durch direkte Destillation ist nicht möglich, da dazu auch bei der Arbeitsweise unter reduziertem Druck Temperaturen benötigt werden, die zu unkontrollierbaren Zersetzungen führen. In Ulmann's Encyclopedia of Industrial Chemistry, 5. Auflage, Band A3, S. 470 (1985) wird ausdrücklich vor der Gefahr von Explosionen gewarnt, wenn man rohe, 3-Nitrobenzaldehyd enthaltende Gemische unter vermindertem Druck destilliert.

Bei dem technisch bisher besten Verfahren zur Herstellung von 2-Nitrobenzaldehyd wird deshalb ein 2-Nitrobenzaldehyd enthaltendes Isomerengemisch zunächst in die entsprechenden Acetale überführt, dann wird aus dem Acetalgemisch 2-Nitrobenzaldehydacetal destillativ abgetrennt und abschließend das abgetrennte 2-Nitrobenzaldehydacetal unter Freisetzung von 2-Nitrobenzaldehyd zurückgespalten (s. DE-OS 3212069). Dieses Verfahren liefert ausreichend reinen 2-Nitrobenzaldehyd, jedoch ist es wegen zweier zusätzlicher Reaktionsstufen (Acetalbildung und Acetalspaltung) aufwendig.

Es wurde jetzt ein verbessertes Verfahren zur Trennung von Nitrobenzaldehyd-Isomerengemischen gefunden, daß dadurch gekennzeichnet ist, daß man Nitrobenzaldehyd-Isomerengemische in Gegenwart von monomeren und/oder polymeren aromatischen Aminen und/oder Phenolen und/oder N- und S-haltige Phenothiazinen bei Sumpftemperaturen von maximal 200°C destilliert.

In das erfindungsgemäße Verfahren können beliebige Nitrobenzaldehyd-Isomerengemische eingesetzt werden, z.B. solche, die 2 oder 3 Isomere enthalten. Diese Gemische können von beliebiger Herkunft sein. Vorzugsweise werden Nitrobenzaldehyd-Isomerengemische eingesetzt, wie sie bei der Nitrierung von Benzaldehyd anfallen. Solche Gemische enthalten typischerweise 19 bis 23 Gew.-% 2-Nitrobenzaldehyd, 75 bis 80 Gew.-% 3-Nitrobenzaldehyd und 1 bis 3 Gew.-% 4-Nitrobenzaldehyd. Gegebenenfalls können solche Gemische noch weitere Komponenten enthalten, z.B. 1 bis 5 Gew.-% Wasser und/oder Spuren von Schwefelsäure, Benzaldehyd, Nitrobenzol und/oder Nitrobenzoesäuren.

Vorzugsweise destilliert man beim erfindungsgemäßen Verfahren 2-Nitrobenzaldehyd oder 2- und 3-Nitrobenzaldehyd nacheinander ab.

Die erfindungsgemäß einzusetzenden monomeren und/oder polymeren aromatischen Amine und/oder Phenole, im folgenden auch als Stabilisatoren bezeichnet, können z.B. in Mengen von 0,5 bis 2 Gew.-%, bezogen auf eingesetztes Nitrobenzaldehyd-Isomerengemisch, eingesetzt werden. Höhere Einsatzmengen sind möglich, bringen aber keine weiteren Vorteile. Stabilisatorzusätze von weniger als 0,5 Gew.-% wirken im allgemeinen nicht ausreichend.

Als Stabilisatoren einsetzbare aromatische Amine sind z.B. Pyridin, Diphenylamin, Dihydrochinolin und Naphthylamin und deren Derivate, z.B. solche Derivate, die Phenyl-, C₁-C₄-Alkyl und/oder C₁-C₄-Acylgruppen enthalten. Substituierte und unsubstituierte aromatische Amine können gegegenenfalls polymerisiert sein. Als Einzelbeispiele seien genannt: Pyridin, 2-Phenylpyridin, polymerisiertes Trimethyldihydrochinolin, N-Phenyl-α-naphthylamin und acetyliertes Diphenylamin.

Als Stabilisatoren einsetzbare Phenole kommen z.B. Kresole und 2,2'-Methylenbisphenole und deren Derivate in Frage, z.B. solche Derivate, die geradkettige, verzweigte und/oder cyclische Alkylreste mit bis zu 8 C-Atomen enthalten. Substituierte und unsubstituierte Phenole können gegebenenfalls polymerisiert sein.

Als Einzelbeispiele seien genannt: 2,2'-Methylen-bis-(4-methyl-6-tert.-butylphenol), Di-tert.-butylkresol und 2,2'-Methylen-bis-(4-methyl-6-cyclohexylphenol).

Man kann auch S- und/oder N-haltige, vorzugsweise sterisch gehinderte aromatische Phenole oder N- und S-haltige Phenothiazine einsetzen.

In das erfindungsgemäße Verfahren einsetzbare Stabilisatoren sind z.B. in Form verschiedener Vulkanox® -Typen im Handel erhältlich.

Vorzugsweise führt man das erfindungsgemäße Verfahren bei Sumpftemperaturen im Bereich 150 bis 190°C und bei Kopftemperaturen von 110 bis 135°C durch. Das bedeutet, daß man die Destillation bei vermindertem Druck durchführen muß, beispielsweise bei 1 bis 30 mbar.

Die Destillation kann man kontinuierlich oder diskontinuierlich durchführen. Zweckmäßigerweise verwendet man dabei eine Kolonne, beispielsweise eine Füllkörperkolonne.

Man kann die zuzusetzenden Stabilisatoren beispielsweise dem aufgeschmolzenen, einzusetzenden Nitrobenzaldehyd-Isomerengemisch zufügen. Die Stabilisatoren verbleiben nach der Destillation im Sumpf und können gegebenenfalls wieder verwendet werden.

Auf die erfindungsgemäße Weise kann man als Zwischenprodukt für Pharmazeutika geeignete 2- und 3- Nitrobenzaldehyde auf einfache und vorteilhafte Weise erhalten ohne die Notwendigkeit der Überführung in eine Acetalzwischenstufe und ohne Risiko von unkontrollierbaren Zersetzungen.

Dies ist außerordentlich überraschend, denn die einzusetzenden Stabilisatoren werden sonst als Antioxidantien in der Kautschukchemie eingesetzt. Bei der Destillation von Nitrobenzaldehyden sind jedoch nicht Oxidationsreaktionen der Anlaß zu unkontrollierbaren Zersetzungen, sondern eine Disproportionierung der Aldehydgruppen.

Durch eigene Untersuchungen wurde festgestellt, daß die Durchführung der Destillation von Nitrobenzaldehyd-Isomerengemischen unter Stickstoff das Risiko von unkontrollierbaren Zersetzungen nicht mindert.

### Beispiel 1:

47 g eines Gemisches, das 9,74 g 2-Nitrobenzaldehyd, 2 Gew.-% Wasser und daneben noch 3- und 4-Nitrobenzaldehyd enthielt wurde mit 1,0 g styrolisiertem Diphenylamin versetzt und in einer Destillationsapparatur bei 22 mbar und 180°C Sumpftemperatur (Badtemperatur) unter Rühren destilliert. Im Destillat wurden 9,35 g 2-Nitrobenzaldehyd analysiert (GC). Das entspricht 96 Gew.-% des Einsatzes.

### Beispiel 2:

(zur Erläuterung des beim erfindungsgemäßen Verfahrens nicht mehr bestehenden Risikos von unkontrollierbaren Zersetzungen)

Ein Isomerengemisch, das 2-, 3- und 4-Nitrobenzaldehyd und 2,5 Gew.-% Wasser enthielt wurde in ein Probenrohr eingewogen und im Wärmeflußkalorimeter vermessen. Bei einer Heizungsrate von 3 K/min zeigte sich eine deutliche Exothermie, beginnend bei ca. 180°C und mit einem Maximum bei ca. 210°C.

Die Wiederholung dieser Messung mit dem gleichen Isomerengemisch, das jedoch 2 Gew.-% polymeres Trimethyldihydrochinolin enthielt ergab, daß nunmehr die Exothermie bei ca. 230°C begann und das Maximum bei ca. 280°C aufwies.

## Patentansprüche

1. Verfahren zur Trennung von Nitrobenzaldehyd-Isomerengemischen, dadurch gekennzeichnet, daß man Nitrobenzaldehyd-Isomerengemische in Gegenwart von monomeren und/oder polymeren aromatischen Aminen und/oder Phenolen und/oder N- und S-haltigen Phenothiazinen bei Sumpftemperaturen von maximal 200°C destilliert.

2. Verfahren nach Anspruch 1, dadurch gekennzeichnet, daß man Nitrobenzaldehyd-Isomerengemische einsetzt, die 2 oder 3 Isomere enthalten.

3. Verfahren nach Ansprüchen 1 und 2, dadurch gekennzeichnet, daß man Nitrobenzaldehyd-Isomerengemische einsetzt, wie sie bei der Nitrierung von Benzaldehyd anfallen.

4. Verfahren nach Ansprüchen 1 bis 3, dadurch gekennzeichnet, daß man 0,5 bis 2 Gew.-% monomere und/oder polymere, aromatische Amine und/oder Phenole, bezogen auf eingesetztes Nitrobenzaldehyd-Isomerengemisch, einsetzt.

5. Verfahren nach Ansprüchen 1 bis 4, dadurch gekennzeichnet, daß man als aromatische Amine gegebenenfalls polymerisiertes Pyridin, Diphenylamin, Dihydrochinolin oder Naphthylamin oder deren Derivate einsetzt, die Phenyl-, C₁-C₄-Alkyl- oder C₁-C₄-Acylgruppen enthalten.

6. Verfahren nach Ansprüchen 1 bis 5, dadurch gekennzeichnet, daß als Phenole, Kresole und 2,2'-Methylen-bis-phenole oder deren Derivate eingesetzt werden, die geradkettige, verzweigte und/oder cyclische Alkylreste mit bis zu 8 C-Atomen enthalten.

7. Verfahren nach Ansprüchen 1 bis 6, dadurch gekennzeichnet, daß man bei Sumpftemperaturen im Bereich 150 bis 190°C, bei Kopftemperaturen im Bereich 110 bis 135°C und bei Drucken im Bereich 1 bis 30 mbar arbeitet.

## Claims

1. Process for separating mixtures of nitrobenzaldehyde isomers, characterized in that mixtures of nitrobenzaldehyde isomers are distilled at bottom temperatures of at most 200°C in the presence of monomeric and/or polymeric aromatic amines and/or phenols and/or N- and S-containing phenothiazines.

2. Process according to Claim 1, characterized in that mixtures of nitrobenzaldehyde isomers are used which contain 2 or 3 isomers.

3. Process according to Claims 1 and 2, characterized in that mixtures of nitrobenzaldehyde isomers are used as arise in the nitration of benzaldehyde.

4. Process according to Claims 1 to 3, characterized in that 0.5 to 2% by weight of monomeric and/or polymeric aromatic amines and/or phenols are used, based on the mixture of nitrobenzaldehyde isomers used.

5. Process according to Claims 1 to 4, characterized in that the aromatic amines used are polymerized or unpolymerized pyridine, diphenylamine, dihydroquinoline or naphthylamine or derivatives thereof which contain phenyl, C₁-C₄-alkyl or C₁-C₄-acyl groups.

6. Process according to Claims 1 to 5, characterized in that the phenols used are cresols and 2,2'-methylene-bis-phenols or derivatives thereof which contain straight-chain, branched and/or cyclic alkyl radicals having up to 8 C atoms.

7. Process according to Claims 1 to 6, characterized in that bottom temperatures in the range 150 to 190°C, top temperatures in the range 110 to 135°C and pressures in the range 1 to 30 mbar are employed.

## Revendications

1. Procédé pour la séparation de mélanges d'isomères de nitrobenzaldéhyde, caractérisé en ce qu'on soumet des mélanges d'isomères de nitrobenzaldéhyde à une distillation en présence de phénols et/ou de phénothiazines à teneurs N et S et/ou d'amines aromatiques, monomères et/ou polymères, à des températures maximales de bas de colonne de 200°C.

2. Procédé selon la revendication 1, caractérisé en ce qu'on met en oeuvre des mélanges d'isomères de nitrobenzaldéhyde qui contiennent l'isomère 2 ou l'isomère 3.

3. Procédé selon la revendication 1 ou 2, caractérisé en ce qu'on met en oeuvre des mélanges d'isomères de nitrobenzaldéhyde tels qu'on les obtient lors de la nitration du benzaldéhyde.

4. Procédé selon les revendications 1 à 3, caractérisé en ce qu'on met en oeuvre des phénols et/ou des amines aromatiques monomères et/ou polymères à concurrence de 0,5 à 2% en poids rapportés au mélange d'isomères de nitrobenzaldéhyde mis en oeuvre.

5. Procédé selon les revendications 1 à 4, caractérisé en ce qu'on met en oeuvre, à titre d'amines aromatiques, de la pyridine, de la diphénylamine, de la dihydroquinoléine ou de la naphtylamine éventuellement polymérisées ou leurs dérivés qui contiennent des groupes phényle, alkyle en C₁-C₄ ou acyle en C₁-C₄.

6. Procédé selon les revendications 1 à 5, caractérisé en ce qu'on met en oeuvre, à titre de phénols, des crésols et des 2,2'-méthylène-bis-phénols ou leurs dérivés qui contiennent des radicaux alkyle à chaînes droites, à chaînes ramifiées et/ou cycliques contenant jusqu'à 8 atomes de carbone.

7. Procédé selon les revendications 1 à 6, caractérisé en ce qu'on travaille à des températures de bas de colonne dans le domaine de 150 à 190°C, à des températures de tête dans le domaine de 110 à 135°C et sous des pressions dans le domaine de 1 à 30 mbar.
